# EUROPÄISCHE PATENTSCHRIFT

(11) **EP 0 968 277 B1**
(45) Veröffentlichungstag und Bekanntmachung des Hinweises auf die Patenterteilung: **17.06.2009**
(21) Anmeldenummer: 98913653.6
(22) Anmeldetag: 05.03.1998
(51) Int. Cl.: C12N 7/02, C12N 15/86, A61K 48/00

(54) **FILTRATIONSVERFAHREN ZUR TRENNUNG VON VIREN**
FILTRATION METHOD FOR SEPARATING VIRUSES
PROCEDE DE FILTRATION DESTINE A LA SEPARATION DE VIRUS

(30) Priorität: 06.03.1997 DE 19709186
(43) Veröffentlichungstag der Anmeldung: 05.01.2000
(73) Patentinhaber: MediGene AG, 82152 Martinsried (DE)
(72) Erfinder: BOGEDAIN, Christoph, D-80333 München (DE); MAASS, Gerhard, D-82377 Penzberg (DE); HÖRER, Markus, D-82152 Planegg (DE)
(74) Vertreter: Bösl, Raphael Konrad
(86) Internationale Anmeldenummer: PCT/EP1998/001257
(87) Internationale Veröffentlichungsnummer: WO 1998/039420

(56) Entgegenhaltungen:
- WO-A-89/08701
- WO-A-95/06743
- WO-A-96/26742
- WO-A-97/08298
- DE-A- 1 951 800
- DE-C- 471 105
- A.MAYR ET AL: "Virologische Arbeitsmethode" 1974 , VEB GUSTAV FISCHER VERLAG XP002070257 siehe Seite 258 - Seite 259
- BINIE A. VER ET AL: "Efficient filtration and sizing of viruses with membrane filters" JOURNAL OF VIROLOGY, Bd. 2, Nr. 1, 1968, Seiten 21-25, XP002070256
- MUZYCZKA N: "USE OF ADENO-ASSOCIATED VIRUS AS A GENERAL TRANSDUCTION VECTOR FOR MAMMALIAN CELLS" CURRENT TOPICS IN MICROBIOLOGY AND IMMUNOLOGY, Bd. 158, 1992, Seiten 97-129, XP000647517

## Beschreibung

Die Erfindung betrifft ein Verfahren zur quantitativen Trennung von Viren unterschiedlicher Größe, wobei 1-10 ml pro 1 cm² Filteroberfläche einer Virus-enthaltenden Lösung filtriert wird, die Virus-enthaltende Lösung über eine oder mehrere Filtermembranen filtriert wird, der Größenunterschied der zu trennenden Viren mindestens 40 nm beträgt, die Viren eine im wesentlichen kugelige Form, insbesondere mit Ikosaeder-Symmetrie besitzen, und eine Titerreduktion der abzutrennenden Viren von mehr als log9 erreicht wird.

Das ursprüngliche Ziel der Gentherapie war es, genetische Erkrankungen durch geeignete genetische Veränderungen von Körperzellen zu heilen. Heutzutage wird der Begriff Gentherapie auf genetisch veränderte Zellen ausgedehnt, die auch für die Heilung von nicht genetisch bedingten Erkrankungen, wie beispielsweise virale Erkrankungen, therapeutisch eingesetzt werden können.

Für die genetische Veränderung von therapeutisch wirksamen Zellen sind geeignete Verfahren zur Übertragung der Nukleinsäure, DNA oder RNA, notwendig, die die genetische Veränderung der Zelle verursachen. Die zu übertragenen. Nukleinsäuren werden häufig auch als sogenannte Transgene bezeichnet, selbst wenn sie nicht die Funktionen eines Gens ausüben, z. B. anti-sense Nukleinsäuren. Neben dem direkten Gentransfer von sogenannten nackten Nukleinsäuren haben sich auch genetisch veränderte Viren für den Gentransfer als geeignet erwiesen. Zur Zeit werden u. a. Retroviren, Adenoviren oder Adeno-assoziierte Viren (AAV) genetisch verändert, damit sie als Träger (viraler Vektor) des oder der Transgene für den Gentransfer verwendet werden können. Ein wesentlicher Gesichtspunkt bei der Entwicklung geeigneter viraler Vektoren sind Sicherheitsaspekte bei der Anwendung dieser Vektoren in der Gentherapie. Daher werden im allgemeinen replikationsdefiziente Viren entwickelt, also Viren, die zwar eine Zelle befallen und das oder die Transgene auf die Zelle übertragen können, sich in dieser Zelle jedoch nicht selbst vermehren können. Dies wird beispielsweise dadurch erreicht, daß man für die Virusvermehrung wichtige Gene, beispielsweise die für Strukturproteine kodierenden Gene, deletiert und gegebenenfalls an deren Stelle das oder die Transgene einbaut. Bei der Herstellung größerer für die gentherapeutische Verwendung geeigneter Mengen von nicht vermehrungsfähigen Viren sind sogenannte Helferviren notwendig, die den Defekt bei einem nicht vermehrungsfähigen Virus in der Zelle kompensieren. Die folgenden Beispiele zu retroviralen Vektoren und Adeno-assoziierten Vektroren sollen das allgemeine Prinzip näher erläutern:

Replikationsdefiziente retrovirale Vektoren leiten sich von Wildtyp-Retroviren ab, die eine eigene Familie von eukaryotischen Viren darstellen, deren genetisches Material (Strukturgene und regulatorische Gene) aus einzelsträngiger RNA besteht. Die Viren bestehen aus sphärischen, umhüllten Viruspartikeln mit einem Durchmesser von ca. 80-120 nm und einer inneren Kapsel, die zwei Kopien der genomischen RNA in Form eines Ribonucleoproteins enthält. Für die Herstellung retroviraler Vektoren werden beispielsweise ein oder mehrere Strukturgene (gag, pol und/oder env) durch das oder die Transgene ersetzt. Die am 5'- und am 3'-Ende noch vorhandenen LTR-Regionen ("long terminal repeats") enthalten als cis-aktive Elemente regulatorische Sequenzen, wie z. B. einen Promotor, Polyadenylierungssignale und für die genomische Integration notwendige Sequenzen. Der retrovirale Vektor kann daher lediglich die LTR-Regionen enthalten, die das oder die Transgene begrenzen. Für die Vermehrung eines replikationsdefizienten retroviralen Vektors wird daher beispielsweise ein Helfervirus, das ein oder mehrere der oben genannten retroviralen Strukturgene enthält, benötigt und so das oder die deletierten Strukturgene komplementiert (Whartenby, K. A. et al. (1995) Pharmac. Ther., 66, 175-190).

Replikationsdefiziente Adeno-assoziierte Virusvektoren leiten sich vom Wildtyp-AAV ab, das ein nicht-autonom replizierender Vertreter der Parvoviren ist und einen einzelsträngigen DNA Virus mit einem Durchmesser von ca. 25 nm darstellt. Bis heute kann man die serologisch unterschiedlichen Typen AAV-1, AAV-2, AAV-3, AAV-4 und AAV-5 unterscheiden. AAV-Viren können entweder in das Genom der Wirtszelle integrieren oder in Anwesenheit eines Helfervirus in den Wirtszellen replizieren. Als mögliche Helferviren wurden zuerst Adenoviren gefunden. Adenoviren bilden beispielsweise in Vertebraten eine Gruppe von mehr als 80 serologisch unterscheidbare Serotypen und enthalten eine äußere, idosaederförmige Proteinhülle (Capsid) und einen inneren DNA-Protein-Zentralkörper (Core). Das Capsid ist wiederum aus 252 Untereinheiten, sogenannte Capsomere, zusammengesetzt. Die Adenoviren besitzen im allgemeinen einen Durchmesser von ca. 70-90 nm und enthalten eine doppelsträngige, lineare DNA als genetisches Material, an deren 5'- und 3'-Enden ITR-Regionen ("inverted terminal repeats") sitzen. Die Replikation von AAV (lytische Phase) benötigt nun insbesondere die Expression früher adenoviraler Gene, wie z. B. das E1a-, E1b-, E2a- und E4-Gen und die VA-RNA (Kotin, R. M. (1994) Human Gene Therapy, 5, 793-801). Es eignen sich jedoch auch andere Helferviren, wie z. B. Herpesviren, eine Gruppe human- und tierpathogener doppelsträngiger DNA-Viren mit einem Durchmesser von ca. 120-200 nm, wobei das Capsid eine Ikosaederstruktur aufweist und aus 162 Capsomeren aufgebaut ist. Die Herpesviren lassen sich in drei Unterfamilien unterteilen, wobei zu den Alphaherpesvirinae z. B. Herpes simplex-Viren (HSV) Typ 1 und Typ 2 gehören, zu den Betaherpesvirinae z. B. das Cytomegalievirus (CMV) und zu den Gammaherpesvirinae z. B. das Epstein-Barr-Virus (EBV).

Analog den retroviralen Vektoren können für die Herstellung Adeno-assoziierter Vektoren beispielsweise ein oder mehrere für die Replikation notwendige rep-Gene (z. B. rep 40, rep 52. rep 68 und/oder rep 78) oder die für die Capsidstruktur notwendigen cap-Gene (z. B. VP-1, VP-2 und/oder VP-3) durch das oder die Transgene ersetzt werden. Die am 5'- und am 3'-Ende noch vorhandenen ITR-Regionen sind als cis-aktive Elemente zur Verpackung des Transgens in infektiöse rekombinante AAV-Partikel sowie für die DNA-Replikation des rekombinanten AAV-Genoms erforderlich (Kotin, R. M. (1994), supra).

Ein vorteilhaftes Verfahren zur Herstellung größerer Mengen an rekombinanten AAV-Partikeln ist die Kotransfektion einer eukaryotischen Zelle mit zwei rekombinanten AAV-Plasmiden und einem Helfervirus (Chiorini, J. A. et al. (1995) Human Gene Therapy, 6. 1531-1541). Der erste rekombinante AAV-Vektor enthält das oder die Transgene, welche von den beiden ITR-Regionen begrenzt werden. Das zweite rekombinante AAV-Plasmid enthält die AAV-Gene, die für die Herstellung der Partikel benötigt werden (rep- und cap-Gene). Das Fehlen funktioneller ITR-Regionen im zweiten Vektor verhindert die Verpackung der rep- und cap-Gene in AAV-Partikel und damit die Entstehung von unerwünschtem Wildtyp-AAV. Anschließend werden Säugerzellen, beispielsweise COS-7 Zellen, die sowohl für die rekombinanten AAV-Vektoren als auch für das Helfervirus, beispielsweise Adenovirus, permissiv sind, d.h. die Voraussetzungen zur Infektion und zur Replikation bieten, mit den beiden rekombinanten AAV-Vektoren und dem Helfervirus transfiziert. Als Helfervirus ist insbesondere das Adenovirus geeignet, da es ein breites Spektrum von Zielzellen infizieren und in den Zellen selbst replizieren kann. Bei der Kultivierung der transfizierten Zellen erfolgt die Expression der AAV-Nichtstrukturproteingene, der AAV-Strukturproteingene, die Replikation von Transgen-DNA und die Verpackung und der Zusammenbau der rekombinanten AAV-Partikel (rAAV-Partikel). Die rAAV-Partikel enthalten das oder die Transgene, beidseitig flankiert von den ITR-Regionen, in Form einzelsträngiger DNA. Gleichzeitig repliziert das Helfervirus in diesen Zellen, was bei Adenoviren als Helferviren nach einigen Tagen im allgemeinen mit der Lyse und dem Tod der infizierten Zellen endet. Die entstandenen Viren (Adenoviren und rAAV-Partikel) werden hierbei teilweise in den Zeltkulturüberstand freigesetzt oder verbleiben in den lysierten Zellen. Daher werden im allgemeinen für eine im wesentlichen vollständige Freisetzung der Viren die Zellen mit dem Fachmann bekannten Zellaufschlußmethoden, wie z. B. abwechselndes Einfrieren und Auftauen oder mit Hilfe einer enzymatischen Hydrolyse beispielsweise mit Trypsin (Chiorini, J. A. et al. (1995), supra), aufgeschlossen.

Ein wesentlicher Nachteil bei der Herstellung von viralen Vektoren mit Hilfe von Helferviren ist das Entstehen einer Mischpopulation aus rekombinanten Viruspartikeln und Helferviren, die weiter gereinigt werden muß. Insbesondere sind Adenovirus-Kontaminationen bei der Verwendung von viralen Vektoren in der Gentherapie wegen der potentiellen Pathogenität zu vermeiden bzw. zu minimieren.

Übliche Verfahren zur Abreicherung bzw. Abscheidung von beispielsweise Adenoviren aus Mischpopulationen mit beispielsweise AAV-Partikeln sind die Dichtegradientenzentrifugation. Hitzeinaktivierung oder eine Kombination beider Methoden. Die Wirkungsweise der Dichtegradientenzentrifugation beruht in diesem Fall auf einem geringfügigen Unterschied in der Dichte der Adenoviren (1,35 g/cm³) und der AAV-Partikel (1,41 g/cm³), der beispielsweise in der CsCl-Dichtegradientenzentrifugation ausgenutzt werden kann. Aufgrund des geringen Dichteunterschiedes der Viren und der ca. 10- bis 100-fach höheren Menge an Adenoviren als an AAV-Partikeln, liegen die Bereiche, in denen sich die AAV-Partikel und die Adenoviren nach der CsCl-Dichtegradientenzentrifugation befinden, sehr nahe, so daß eine quantitative Trennung der beiden Viren durch einen oder mehrere Dichtegradienten nicht möglich ist. Zudem muß sich die Mischpopulation in einem relativ geringen Volumen befinden, da die Dichtegradientenzentrifugation nur mit relativ wenig Volumen durchgeführt werden kann. Eine routinemäßige Anwendung im industriellen Maßstab ist daher technisch und finanziell nicht möglich. Zudem kann eine quantitative Abscheidung von Adenoviren aus der Mischpopulation nicht erreicht werden.

Die Wirkungsweise der Hitzeinaktivierung beruht auf einer unterschiedlichen Thermostabilität von beispielsweise Adenoviren und AAV-Partikeln. Die Erwärmung einer Mischpopulation aus Adenoviren und AAV-Partikeln auf 56-65 °C führt zu einer mehr oder weniger selektiven Hitzeinaktivierung der Adenoviren unter nur geringem Aktivitätsverlust der AAV-Partikel. Nachteil dieser Methode ist jedoch, daß bei der gentherapeutischen Anwendung die noch in der Präparation vorhandenen denaturierten Adenovirus-Proteine eine starke zelluläre Immunantwort beim Patienten hervorrufen können (Smith, C. A. et al. (1996) J. Virol., 70, 6733-6740).

Es ist auch bekannt, daß zur Abtrennung viraler Kontaminationen aus biopharmazeutischen Produkten, die frei von Viren sein müssen, Filtermembranen mit einer Porengröße, die eine Abscheiderate von Partikeln im Nanometerbereich gewährleistet, eingesetzt werden können (Scheiblauer, H. et al. (1996), Jahrestagung der Gesellschaft für Virologie, Abstract Nr. P54). Scheiblauer et al. (1996, supra) weist zwar darauf hin, daß die Viren aufgrund ihrer Größe zurückgehalten werden, von einer quantitativen Trennung verschiedener Viren wurde jedoch nicht berichtet.

Die Druckschrift DE 1951800 A (Zander Helmut, April 1971) offenbart ein präparatives Reinigungsverfahren im Herstellungsgang von Impfstoffen gegen Viruskrankheiten, bei dem ein oder mehrere Reinigungsschritte mit Hilfe von Controlliert Porösem Glas (CPG) in den Herstellungsgang von Impfstoffen eingeführt sind.

Aufgabe der vorliegenden Erfindung ist es daher, ein Verfahren bereitzustellen, bei dem unterschiedliche in einer Mischpopulation enthaltene Viren voneinander getrennt werden können und das auch bei größeren Volumen angewandt werden kann.

Es wurde nun überraschenderweise gefunden, daß eine im wesentlichen quantitative bzw. für die Verwendung in der Gentherapie ausreichende Trennung von Viren unterschiedlicher Größe durch eine oder mehrere Filtrationen einer Virus-enthaltenden Lösung auf eine einfache und auch im industriellen Maßstab anwendbare Weise erreicht wird.

Ein Gegenstand der vorliegenden Erfindung ist daher ein Verfahren zur quantitativen Trennung von Viren unterschiedlicher Größe, wobei 1-10 ml pro 1 cm² Filteroberfläche einer Virus-enthaltenden Lösung filtriert wird, die Virus-enthaltende Lösung über eine oder mehrere Filtermembranen filtriert wird, der Größenunterschied der zu trennenden Viren mindestens 40 nm beträgt, die Viren eine im wesentlichen kugelige Form, insbesondere mit Ikosaeder-Symmetrie besitzen, und eine Titerreduktion der abzutrennenden Viren von mehr als log9 erreicht wird.

Vorteilhafterweise bestehen die Filtermembranen aus Polyvinylidenfluorid, Polytetrafluormethylen, Polypropylen, modifiziertes oder nicht-modifiziertes Polyethersulfon, Zelluloseacetat, Zellulosenitrat, Polyamid oder regenerierte Zellulose, insbesondere Cuprammonium-regenerierte Zellulose, vorzugsweise aus Polyvinylidenfluorid. Derartige Membranen mit einer Porengröße, welche die Abscheidung von Partikeln mit einer Größe von ca. 40-400 nm erlauben, sind beispielsweise die Ultipor Membranen der Fa. Pall GmbH, 63303 Dreieich, mit einer Porenweite von ca. 50 nm oder ca. 100 nm, die Asahi Chemical's Bemberg Microporous Membranen der Fa. Asahi Chemical Industry Ltd., Tokio, Japan, mit einer Porenweite von ca. 15 nm, ca. 35 nm oder ca. 72 nm, oder entsprechende Membranen der Fa. Sartorius AG, 37075 Göttingen oder Schleicher und Schuell GmbH, 37582 Dassel. Im allgemeinen haben die Filtermembranen gemäß der vorliegenden Erfindung eine Porenweite, von ca. 25-60 nm. Die Auswahl eines Filters mit geeigneter Porenweite richtet sich in erster Linie nach der Größe der zu trennenden Viren, die im allgemeinen einen Durchmesser von bis zu ca. 250 nm, vorzugsweise bis zu ca. 120 nm, insbesondere bis zu ca. 60 nm besitzen. Ein weiteres Auswahlkriterium bei der Auswahl eines Filters mit geeigneter Porenweite ist der Größenunterschied der zu trennenden Viren, der im allgemeinen mindestens ca. 5 nm, vorzugsweise mindestens ca. 10 nm, insbesondere mindestens ca. 20 nm, vor allem mindestens ca. 30 nm und am meisten bevorzugt ca. 40 nm betragen soll. Anhand der Größe der zu trennenden Viren und dem daraus resultierenden Größenunterschied kann der Fachmann einen Filter mit einer zur Trennung der zu trennenden Viren geeigneten Porengröße auswählen. Die Wirksamkeit des oder der ausgewählten Filter kann dann leicht anhand von einfachen Filtrationsexperimenten routinemäßig getestet werden.

So eignet sich beispielsweise ein Filter mit einer Porenweite, die eine Titerreduktion von Viren mit einem Durchmesser von über ca. 50 nm ermöglicht, vorzugsweise die oben bereits erwähnte Pall-Ultipor VF DV50 Membran oder die Asahi-Planova 35-Membran, besonders vorteilhaft zur Trennung von rAAV-Partikeln und Adenoviren. Insbesondere diese Filter ermöglichen nicht nur die im wesentlichen quantitative Abscheidung infektiöser Adenoviren aus der Mischpopulation, sondern auch eine hohe Ausbeute an rAAV-Partikeln von ca. 70-90%.

Die Begriffe "Porenweite", "Abscheiderate", "Wirkgröße" oder "Porenweite, die eine Abscheidung von Partikeln mit einer bestimmten Mindestgröße erlauben", die von verschiedenen Filterherstellern verwendet werden, sind im Sinne der vorliegenden Erfindung äquivalente Begriffe.

Der Begriff Virus bzw. Viren umfaßt im Sinne der vorliegenden Erfindung nicht nur natürlich vorkommende Viren oder gentechnisch veränderte, sogenannte rekombinante Viren, sondern auch Virus-Partikel, d. h. infektiöse wie auch nicht-infektiöse Viren, virus-ähnliche Partikel ("VLP"), wie z. B. Papillomavirus-ähnliche Partikel gemäß WO 96/11272. und Capside, die Nukleinsäuren enthalten, aber auch leer sein können und Teile davon, insbesondere eine oder mehrere, vorzugsweise mehrere Untereinheiten bzw. Capsomere, vor allem wenn mehrere Capsomere derart assoziiert bzw. verbunden sind, daß sie mindestens ca. 50%, vorzugsweise mindestens ca. 80%, vor allem ca. 90% des Capsids ausmachen. Die Viren haben vor allem eine im wesentlichen kugelige Form, insbesondere mit Ikosaeder-Symmetrie.

Gemäß der vorliegenden Erfindung können die Viren nach ihrer Größe in folgende drei Gruppen eingeteilt werden:

Die erste Gruppe repräsentiert Viren mit einem Durchmesser bis zu ca. 60 nm, die sich im allgemeinen beispielsweise von Flaviviridae mit einem Durchmesser von ca. 40-60 nm, wie z. B. Flavivirus oder Hepatitis C Virus (HCV) ableiten, von Papillomaviren mit einem Durchmesser von ca. 55 nm, wie z. B. die humanen Papillomaviren 16 oder 18 (HPV 16, HPV 18), von Papovaviren mit einem Durchmesser von ca. 45 nm, wie z. B. das Polyomavirus, von Hepadnaviren mit einem Durchmesser von ca. 22-42 nm, wie z. B. das Hepatits B Virus (HBV), von Picornaviren mit einem Durchmesser von ca. 22-30 nm, wie z. B. Hepatits A Virus (HAV) oder Poliovirus, oder von Parvoviren mit einem Durchmesser von ca. 18-26 nm, wie z. B. Adenoassoziierter Virus (AAV) oder rAAV-Partikel mit einem Durchmesser von ca. 25 nm.

Die zweite Gruppe repräsentiert Viren mit einem Durchmesser von ca. 60 nm bis zu ca. 120 nm, die sich im allgemeinen beispielsweise von Influenzaviren mit einem Durchmesser von ca. 80-120 nm ableiten, von Retroviren mit einem Durchmesser von ca. 80-120 nm, wie z. B. Onkoviren, Lentiviren, wie beispielsweise HIV-1 oder HIV-2. Spumaviren oder HTLV-Viren, von Adenoviren mit einem Durchmesser von ca. 65-90 nm, von Reoviren mit einem Durchmesser von ca. 60-80 nm, wie z. B. Coltivirus oder Rotavirus, oder von Togaviren mit einem Durchmesser von ca. 60-70 nm.

Die dritte Gruppe repräsentiert Viren mit einem Durchmesser von ca. 120 nm bis zu ca. 250 nm, die sich im allgemeinen beispielsweise von Paramyxoviren mit einem Durchmesser von ca. 150-250 nm ableiten oder von Herpesviren mit einem Durchmesser von ca. 120-200 nm, wie z. B. HSV-1 oder HSV-2, CMV oder EBV.

Gemäß der vorliegenden Erfindung lassen sich die Viren aus einer der oben genannten Gruppen besonders vorteilhaft von Viren aus den anderen Gruppen trennen. Es lassen sich aber auch unterschiedliche Viren, die ein und derselben der oben genannten Gruppen angehören, voneinander trennen, sofern ein ausreichender Größenunterschied, vorzugsweise wie oben bereits näher beschrieben, vorhanden ist. Insbesondere lassen sich die Viren aus der ersten Gruppe von den Viren aus der zweiten und/oder dritten Gruppe trennen, vor allem lassen sich AAV-Partikel besonders vorteilhaft von Adenoviren und/oder Herpesviren trennen.

Das erfindungsgemäße Verfahren ermöglicht beispielsweise in besonders vorteilhafter Weise die Abscheidung von Adenoviren sowohl im Falle von z. B. durch eine oder mehrere Dichtegradienten vorgereinigtes als auch von nicht gereinigtem Material aus den Überständen beispielsweise einer rAAV Kultur, wobei die Adenoviren im Retentat verbleiben und die rAAV-Partikel sich im Filtrat befinden. Vorteilhafterweise wird vor der eigentlichen Trennung der gewünschten Viren eine Vorfiltration, wie unten näher beschrieben, durchgeführt.

Eine weitere bevorzugte Ausführungsform des erfindungsgemäßen Verfahrens erstreckt sich daher auf eine Vorreinigung der Virus-enthaltenden Lösung, beispielsweise über einen oder mehrere Dichtegradienten und/oder über eine oder mehrere Vorfiltrationen. Besonders bevorzugt ist eine Vorreinigung mit Hilfe eines oder mehrerer Membranfilter, die die zu trennenden Viren passieren lassen, größere Verunreinigungen jedoch zurückhalten. Durch die Vorreinigung wird im allgemeinen eine Verstopfung des Filters, der die anschließende Trennung der gewünschten Viren bewirkt, durch Bestandteile der Viruskultur, die auch durch Zentrifugation bei niedriger Drehzahl nicht bzw. nicht ausreichend zu entfernen sind, verhindert bzw. erschwert. Hierbei besitzt der Membranfilter zur Vorreinigung beispielsweise von Viren aus der oben genannten ersten Gruppe eine Porenweite von mindestens ca. 60 nm, zur Vorreinigung von Viren aus der ersten und/oder zweiten Gruppe eine Porenweite von mindestens ca. 120 nm bzw. zur Vorreinigung von Viren aus der ersten, zweiten und/oder dritten Gruppe eine Porenweite von mindestens ca. 250 nm. Beispielsweise ist ein Membranfilter mit einer Porenweite von ca. 100 nm, wie z. B. der Ultipor N₆₆-Filter (Pall GmbH, 63303 Dreieich), besonders gut geeignet zur Vorreinigung einer Mischpopulation aus rAAV-Partikeln und Adenoviren.

In einer anderen bevorzugten Ausführungsform wird der pH-Wert der Virus-enthaltenden Lösung mit Hilfe geeigneter Puffer, beispielsweise Tris-Cl-Puffer (Tris(hydroxymethyl)aminomethan), auf ca. 6-10, vorzugsweise auf ca. 7,0-8,5, insbesondere auf ca. 8,0 eingestellt, wodurch beispielsweise die Ausbeuten vorzugsweise an AAV erhöht werden können. Es ist auch besonders bevorzugt, wenn die Virus-enthaltende Lösung zusätzlich Protein bzw. Polypeptid, z. B. in Form von fötalem Kälberserum (FCS) oder Serumalbumin, enthält, wobei die Art bzw. Herkunft des Proteins nicht wesentlich ist. Der Anteil an Protein, z.B. in Form von FCS, beträgt im allgemeinen nicht mehr als ca. 30% (v/v), vorzugsweise ca. 15% (v/v), insbesondere ca. 10% (v/v).

In einer weiteren bevorzugten Ausführungsform des erfindungsgemäßen Verfahrens werden ca. 2-5 ml. insbesondere ca. 3 ml der Virus-enthaltenden Lösung pro ca. 1 cm² Filteroberfläche, vor allem nicht mehr als ca. 2-2,5 ml Lösung pro ca. 1 cm² Filteroberfläche filtriert. Hierdurch wird im allgemeinen eine hohe Ausbeute beispielsweise der rAAV-Partikel bei gleichzeitiger Abscheidung beispielsweise der Adenoviren erreicht. Insbesondere ist es bevorzugt, vor der eigentlichen Trennung Zellbestandteile in der Viruskultur durch Zentrifugation bei niedriger Umdrehungszahl, beispielsweise bis zu ca. der 1000fachen Erdbeschleunigung (1000g), vorzugsweise bis zu ca. 500g, insbesondere bis zu ca. 300g, abzutrennen. Darüberhinaus ist es vorteilhaft, anschließend eine oben bereits näher beschriebene Vorfiltration durchzuführen. Auf diese Weise konnte beispielsweise nach Abtrennung der Zellbestandteile bei ca. 300g und Vorfiltration der Mischpopulation über einen, Membranfilter mit einer Porengröße von ca. 100 nm die Kapazität eines Filters mit einer Porengröße von ca. 50 nm zu 2 ml/cm² Filteroberfläche bestimmt werden. Mit Kapazität im Sinne der vorliegenden Erfindung ist das Volumen gemeint, das pro Flächeneinheit Filteroberfläche eine hohe Ausbeute an den gewünschten Viren gewährleistet.

Die Vorteile des erfindungsgemäßen Verfahrens sind vor allem eine einfache, kostengünstige und daher auch im industriellen Maßstab anwendbare Trennung von Viren unterschiedlicher Größe unter besonders schonenden Bedingungen, wodurch in besonders vorteilhafter Weise eine hohe Ausbeute von gereinigten Viren erhalten wird. Ein weiterer Vorteil der vorliegenden Erfindung ist es, daß die erfindungsgemäß gereinigten Viren beispielsweise direkt als virale Vektoren für die Gentherapie eingesetzt werden können, da sie ausreichend frei von anderen, beispielsweise pathogenen Viren sind. So konnte beispielsweise in einer nach dem erfindungsgemäßen Verfahren gereinigten rAAV-enthaltenden Lösung keine Adenoviren anhand eines Replikationstestes, bei dem die Entstehung eines zytopathischen Effekts bei permissiven Zellen innerhalb eines Zeitraumes von beispielsweise 14 Tagen nach Koinkubation mit einer entsprechenden Lösung auf die Anwesenheit von infektiösen Adenoviren hinweisen würde, nachgewiesen werden. Auch konnten mit Hilfe der PCR-Reaktion und DNA-Hybridisierung eine Reduktion adenoviraler Nukleinsäuren um einen Faktor von mindestens ca. 3 x 10³, vorzugsweise um das ca. 10³-10⁴fache nachgewiesen werden. Der im Filtrat verbleibende geringe Restanteil adenoviraler DNA ist jedoch nicht mit replikationskompetenten Adenoviren assoziiert und somit unschädlich. Die Verringerung adenoviraler Bestandteile, wie z. B. freie Adenovirus-Proteine und subvirale Partikel, war besonders überraschend, da diese Bestandteile durchaus eine unspezifische Immunantwort bei der Anwendung viraler Vektoren in der Gentherapie am Patienten auslösen können und folglich die Therapie in Frage stellen könnten. Die Titerreduktion, d. h. der Faktor der Verminderung des Titers der abzutrennenden Viren, betrug beispielsweise bei Adenoviren gemäß dem erfindungsgemäßen Verfahren mehr als log9, d. h. nach der erfindungsgemäßen Filtration einer Mischpopulation aus rAAV-Partikeln und 10⁹ Adenoviren, wurde kein Adenovirus im Filtrat nachgewiesen.

Die folgenden Beispiele soll die Erfindung näher erläutern, ohne sie darauf zu beschränken:

### Beispiele

### Beispiel 1

Filtration einer Mischpopulation aus rAAV-Partikeln und Adenoviren
1.1 Es wurde eine Mischpopulation von rAAV Typ 2-Partikeln, die als Transgen das für T-Zellen kostimulierende Protein B7-2 kodierende Gen enthalten, und Wildtyp Adenovirus Typ 5 nach der Vorschrift von Chiorini, J. A. et al. (1995, supra) hergestellt, wobei 3 Tage nach der Infektion mit den Adenoviren, die Zellen, die bereits einen starken zytopatischen Effekt zeigen, einschließlich des Überstandes gesammelt wurden. Anschließend wurden die Zellen mit Überstand durch dreimaliges Einfrieren und Auftauen aufgeschlossen. Unlösliche Zellbestandteile wurden durch eine 10minütige Zentrifugation bei 300 g abgetrennt. Anschließend wurde der Anteil an fötalem Kälberserum (FCS) auf 10% eingestellt und zu der Präparation Tris·Cl-Puffer (pH 8,0) bis zu einer Endkonzentration von 100mM gegeben. Die von unlöslichen Zellbestandteilen abgetrennte und eingestellte Präparation wurde mit 2,5 bar auf ein Filtersystem beaufschlagt, das eine Pall-Ultipor N₆₆-Membran (Fa. Pall GmbH. 63303 Dreieich) mit einer Wirkgröße von 0,1 µm als Vorfilter sowie eine Pall-Ultipor VF DV50-Membran zur Trennung der beiden Viren enthält. Auf diese Weise wurden nicht mehr als 2 ml der Präparation pro 1 cm² Filteroberfläche filtriert.
1.2 Ausgangsmaterial war der Zellkulturüberstand von rAAV-Partikeln nach dem Protokoll von Chiorini, J. A. et al. (1995, supra). Drei bis vier Tage nach Infektion der Zellen mit dem Adenovirus wurden die Zellen mitsamt des Überstandes vereinigt. Zu der Präparation wurde Tris-Cl-Puffer (pH 8,0) zu einer Endkonzentration von 30 mM gegeben. Die Suspension wurde zum Aufbrechen der Zellen dreimal eingefroren und aufgetaut. Der Schritt der Aufkonzentration der Viren durch eine CsCl-Dichtegradientenkonzentration wurde im Anschluß an die Filtration durchgerührt.
   Die Präparation wurde auf ein Filtersystem beaufschlagt, das aus einem Vorfilter und aus einem Planova 35-Hohlfaser-Filtrationselement mit einer Wirkgröße von 35 nm besteht. Der Vorfilter wurde mit einem Druck von 1,5 bar, der Planova 35-Filter mit einem Druck von 0,5 bar beaufschlagt. Die Menge des filtrierten Volumens betrug beim Vorfilter höchstens 2,5 ml pro cm² Filteroberfläche und beim Planova 35-Filter höchstens 7 ml pro cm² Filteroberfläche.

### Beispiel 2

### Replikationstest

I. Ein Teil einer rAAV-Präparation wurde wie in Beispiel 1 mit einem Filtersystem, das 5 cm² Filteroberfläche hat, filtriert. Die Filtrate wurden in mehreren Aliquots zu 1,5 ml gesammelt. Ein Kontroll-Aliquot verblieb unfiltriert. Von den filtrierten Aliquots sowie von der unfiltrierten Kontrolle wurden jeweils sieben 1:10-Verdünnungsstufen hergestellt. Für den Replikationstest wurden HeLa-Zellen in 48-Napf-Platten als konfluente Zellschicht angewachsen. Der Überstand wurde aus den Näpfen bis auf 100 µl Restvolumen abgezogen. Von der Verdünnungsreihe eines jeden filtrierten Aliquots sowie von der unfiltrierten Kontrolle wurden jeweils 100 µl in je einen Napf überführt. Nach 3 h Adsorption der Viren an die Zellen wurden 500 µl Vollmedium zugegeben. Über einen Zeitraum von einer Woche wurde täglich kontrolliert, ob sich ein zytopathischer Effekt (CPE) zeigt. Bei Bedarf wurde das Medium gewechselt. Der CPE würde durch die Replikation der Viren in den HeLa-Zellen bewirkt. Letztlich endet die Replikation mit dem Tod der infizierten Zelle und mit der Freisetzung der Nachkommenviren.
Tabelle 1 zeigt ein typisches Ergebnis. Während bei der unfiltrierten Kontrolle bis zur 4. Verdünnungsstufe ein CPE nachweisbar war, war die Zellschicht bei allen untersuchten filtrierten Aliquots nach 7 Tagen intakt. Dies bedeutet bei dem unfiltrierten Material einen Titer infektiöser Adenoviren von größenordnunssmäßig 10⁵/ml, während sich in dem filtrierten Material kein infektiöses Adenovirus mehr befindet. Ein vergleichbarer Titer war im Retentat, also in dem Material, das sich direkt über der Membran befand und gesammelt wurde, nachweisbar.

**Tabelle 1**

| Ausbildung eines zytopathischen Effekts 7 Tage nach Koinkubation von HeLa-Zellen mit filtrierten und unfiltrierten Verpackungsüberständen | | | | | | | | | |
|---|---|---|---|---|---|---|---|---|---|
| **Probe** | **Experiment** | **Verdünnungsstufe** | | | | | | | |
| | | 0 | 10⁻¹ | 10⁻² | 10⁻³ | 10⁻⁴ | 10⁻⁵ | 10⁻⁶ | 10⁻⁷ |
| **Kontrolle** | 1 | + | + | + | + | + | - | - | - |
| | 2 | + | + | + | + | + | - | - | - |
| **Aliquot 1** | 1 | - | - | - | - | - | - | - | - |
| | 2 | - | - | - | - | - | - | - | - |
| **Aliquot 2** | 1 | - | - | - | - | - | - | - | - |
| | 2 | - | - | - | - | - | - | - | - |
| **Aliquot 3** | 1 | - | - | - | - | - | - | - | - |
| | 2 | - | - | - | - | - | - | - | - |
| **Retentat** | 1 | + | + | + | + | + | - | - | - |
| | 2 | + | + | + | + | + | - | - | - |

II. Von einer Adenovirus-Präparation, die mit einer Cäsiumchlorid-Dichtegradientenzentrifugation aufkonzentriert wurde, wurde der Adenovirus-Titer, wie oben beschrieben, bestimmt. Es wurden gemäß dieser Titerbestimmung 10⁸ Adenoviren/ml in MEM-Medium mit 10 % FCS und 50 mM Tris·Cl (pH 8,0) aufgenommen. Von dieser Adenovirus-Suspension wurden 10 ml mit einem in Beispiel 1 beschriebenen Filtersystem behandelt, d. h. es wurden insgesamt 10⁹ infektiöse Adenoviren appliziert. Die resultierenden 10 ml Filtrate wurden auf eine 175 cm²-Kulturfläche mit 80 % konfluenten HeLa-Zellen verbracht. Die Flasche wurde 5 Tage inkubiert. Nach diesem Zeitraum zeigte sich kein CPE. Da die Zellen zu diesem Zeitpunkt konfluent waren und da eine längere Versuchsdauer angestrebt wurde, wurden sie 1:5 gesplittet. Die fünf resultierenden HeLa-Kulturen wurden dann über einen weiteren Zeitraum von 5 Tagen beobachtet. Es zeigte sich nach wie vor kein CPE. Daraus wurde auf eine vollständige Abwesenheit von infektiösen Adenoviren im Filtrat geschlossen.

### Beispiel 3

### Nachweis adenoviraler Nukleinsäuren

### a) RT-PCR-Reaktion

Eine rAAV-Teilpräparation wurde wie in Beispiel 1 geschildert filtriert; ein Teil verblieb zur Kontrolle unfiltriert. Es wurden jeweils 3 x 10⁶ Zellen der Melanomzellinie M3HK auf einer 75 cm²-Zellkulturflasche angewachsen. Vier derartige Flaschen wurden vorbereitet und einer Röntgenstrahlung von 50 Gy ausgesetzt. Die Röntgenstrahlung förderte die rAAV-vermittelte Fremdgenexpression sehr stark. Das Medium wurde jeweils abgezogen und in jede Flasche wurden 1.5 ml Vollmedium ohne Serum gegeben. Zusätzlich wurden dann in eine der Flaschen 0,5 ml des unfiltrierten Kontroll-Überstandes, in zwei der Flaschen jeweils 0,5 ml von zwei filtrierten Aliquots und in eine Flasche Medium als Negativkontrolle gegeben. Nach 3 Tagen Inkubation bei 37°C und 10% CO₂ wurden aus dem Zellmaterial jeder Flasche eine RNA-Präparation (Chomchinski, P. und Sacchi, N., Anal. Biochem. 162, 156-159, 1937) durchgeführt. Die RNA-Präparation wurde einer semiquantitativen RT-PCR-Reaktion unterzogen. Zum Nachweis adenoviraler Transkripte sowie des ubiquitäten ß-Aktin-Transkriptes wurden die von Cotten und Mitarbeitern beschriebenen Primerpaare (Cotten, M., Virology, 205, 254-261, 1994) verwendet.

Während bei der unfiltrierten Kontrolle das adenovirale E1A-Transkript in vier von 5 Verdünnungsstufen nachweisbar war, konnte dieses Transkript in den mit den filtrierten Aliquots sowie bei der Negativkontrolle nicht nachgewiesen werden ß-Aktin-Transkripte waren dagegen in allen Parallelen nachweisbar.

### b) DNA-Hybridisierung

Ausgangsmaterial war eine wie in Beispiel 1 beschriebene filtrierte rAAV-Präparation sowie zur Kontrolle ein unfiltriert belassenener Anteil davon. Zu den filtrierten Aliquots sowie der unfiltrierten Kontrolle wurden Tris·Cl (pH 7,8) bis zu einer Endkonzentration von 0,01 M, EDTA bis zu einer Endkonzentration von 0,005 M und SDS bis zu einer Endkonzentration von 0,5 % sowie Proteinase K bis zu einer Endkonzentration von 20 mg/ml zugegeben und 20 min bei 56°C inkubiert.

50 µl der Proteinase K-behandelten Proben wurden zur Denaturierung in 200 µl 0,5 N NaOH aufgenommen. Hiervon wurden acht 1:10-Verdünnungen in 0,5 N NaOH hergestellt. Zusätzlich wurde als Standard von einer bekannte Menge eines Plasmides mit der zu detektierenden DNA-Sequenz in analoger Weise eine Verdünnungsreihe hergestellt. Die Verdünnungsreihen wurden auf eine Nylonmembran aufgedottet. Die Membran wurde bei 80°C zur kovalenten Bindung der DNA inkubiert und danach einer Southern-Hybridisierung mit einer für Adenovirus-DNA spezifischen markierten Probe unterzogen (Chiorini, J. A. et al. (1995), supra). Als Probe wurde ein Digoxygeninmarkiertes Restriktionsfragment mit dem E1A-Gen des Adenovirus verwendet. Die Adenovirus-E1A-DNA auf der Nylonmembran wurde mittels immunologischem Digoxygenin-Nachweis (Boehringer Mannheim GmbH, Mannheim) sichtbar gemacht. In der kleinsten Verdünnungsstufe des E1A-Kontrollplasmides wurden 2 x 10⁸ einzelsträngige DNA-Moleküle aufgetragen. Die kleinste Verdünnungsstufe der unfiltrierten Kontrolle, bei der Material nachweisbar ist, zeigte absolut etwa 5 x 10⁹ Adenovirus-Genome. Dies würden etwa 1 x 10¹⁰ Genome/ml in der AAV-Präparation entsprechen. Bei den filtrierten Aliquots ist in den meisten Fällen in der kleinsten Verdünnungsstufe ein schwaches Signal zu erkennen, das gegenüber der unfiltrerten Kontrolle etwa um einen Faktor 3 x 10³ bis 1 x 10⁴ schwächer ist und 1 x 10⁷ bis 3 x 10⁷ Genome/ml der AAV-Präparation repräsentieren würde. Die in Beispiel 2 geschilderten Tests zeigten jedoch, daß diese Genome in den filtrierten Aliquots nicht mit infektiösen Partikeln assoziiert, sondern in nicht-infektiöser Form vorliegen. Durch die Filtrationstechnil: wurde eine Abreicherung dieser Adenovirus-Genome um einen Faktor 3 x 10³ bis 1 x 10⁴ erzielt.

### Beispiel 4

### Nachweis des adenoviralen Strukturproteins IX im Western-Blot

Das Fehlen des adenoviralen Strukturproteins IX im Filtrat wurde mit spezifischen Antikörpern im Western-Blot nachgewiesen. Von filtrierten Aliquots sowie von unfiltrierten Kontrollen wie auch von Adenovirus-Präparationen wurden jeweils 2 µl zusammen mit Gelauftragspuffer in eine Spur eines Proteingels geladen. Das Material wurde elektrophoretisch getrennt (Laemmli, U. K., Nature 227, 680-685, 1970) und auf eine Nitrozellulosemembran elektrogeblottet. Der Western-Blot wurde mit dem für das Adenovirus-Strukturprotein IX spezifischen Kaninchen-Antiserum inkubiert. Spezifische Banden wurden mit Standard-Techniken sichtbar gemacht. Es zeigte sich, daß das Strukturprotein IX durch die Filtration abgetrennt wurde.

## Patentansprüche

1. Verfahren zur quantitativen Trennung von Viren unterschiedlicher Größe, **dadurch gekennzeichnet, daß** 1-10 ml pro 1 cm² Filteroberfläche einer Virus-enthaltenden Lösung filtriert wird, die Virus-enthaltende Lösung über eine oder mehrere Filtermembranen filtriert wird, der Größenunterschied der zu trennenden Viren mindestens 40 nm beträgt, die Viren eine im wesentlichen kugelige Form, insbesondere mit Ikosaeder-Symmetrie besitzen, und eine Titerreduktion der abzutrennenden Viren von mehr als log9 erreicht wird.

2. Verfahren nach Anspruch 1, **dadurch gekennzeichnet, daß** die Filtermembran aus Polyvinylidenfluorid, Polytetrafluormethylen, Polypropylen, modifiziertes oder nicht-modifiziertes Polyethersulfon, Zelluloseacetat, Zellulosenitrat, Polyamid, oder regenerierte Zellulose, insbesondere Cuprammonium-regenerierte Zellulose, vorzugsweise aus Polyvinylidenfluorid besteht.

3. Verfahren nach einem der Ansprüche 1 oder 2, **dadurch gekennzeichnet, daß** die Filtermembran(en) eine Porenweite von 25-60 nm besitzt/besitzen.

4. Verfahren nach einem der Ansprüche 1-3, **dadurch gekennzeichnet, daß** die Viren ausgewählt sind aus AAV, Adenoviren oder Herpesviren.

5. Verfahren nach einem der Ansprüche 1-4, **dadurch gekennzeichnet, daß** die Virus-enthaltende Lösung vorgereinigt wird.

6. Verfahren nach einem der Ansprüche 1-5, **dadurch gekennzeichnet, daß** die genannte Vorreinigung über einen oder mehrere Dichtegradienten und/oder über eine oder mehrere Vorfiltrationen erfolgt.

7. Verfahren nach Anspruch 6, **dadurch gekennzeichnet, daß** bei der genannten Vorfiltration ein oder mehrere Membranfilter verwendet werden.

8. Verfahren nach Anspruch 7, **dadurch gekennzeichnet, daß** der oder die Membranfilter zur Vorreinigung einePorenweite von mindestens 250 nm besitzt/besitzen.

9. Verfahren nach einem der Ansprüche 1-8, **dadurch gekennzeichnet, daß** der pH-Wert der Virus-enthaltenden Lösung 6-10, vorzugsweise 7,0-8,5, insbesondere 8,0 beträgt.

10. Verfahren nach einem der Ansprüche 1-9, **dadurch gekennzeichnet, daß** die Virus-enthaltende Lösung zusätzlich Protein enthält.

11. Verfahren nach Anspruch 10, **dadurch gekennzeichnet, daß** die genannte Lösung Protein in Form von fötalem Kälberserum oder Serumalbumin enthält.

12. Verfahren nach Anspruch 10 oder 11, **dadurch gekennzeichnet, daß** der Anteil an Protein nicht mehr als 30% (v/v), vorzugsweise 15% (v/v), insbesondere 10% (v/v) beträgt.

13. Verfahren nach einem der Ansprüche 1-12, **dadurch gekennzeichnet, daß** ca. 2-5 ml Lösung pro 1 cm² Filteroberfläche, insbesondere nicht mehr als 2-2,5 ml Lösung pro 1 cm² filtriert werden.

## Claims

1. A process for the quantitative separation of viruses of different sizes, **characterized in that** 1-10 ml of a virus-containing solution is filtered per 1 cm² of filter surface, the virus-containing solution is filtered through one or more filter membranes, the difference in the sizes of viruses to be separated is at least 40 nm, the viruses are of an essentially spherical shape, in particular having an icosahedron symmetry, and **in that** a reduction in titer of the viruses to be separated of more than 9 logs is achieved.

2. The process of claim 1, **characterized in that** the filter membrane consists of polyvinylidene fluoride, polytetrafluoromethylene, polypropylene, modified or unmodified polyether sulfone, cellulose acetate, cellulose nitrate, polyamide, or regenerated cellulose, in particular cuprammonium-regenerated cellulose, preferably polyvinylidene fluoride.

3. The process according to any one of claims 1 or 2, **characterized in that** the filter membrane(s) has/have a pore size of 25- 60 nm.

4. The process according to any one of claims 1-3, **characterized in that** the viruses are selected from AAV, Adenoviruses or Herpesviruses.

5. The process according to any one of claims 1-4, **characterized in that** the virus-containing solution is prepurified.

6. The process according to any one of claims 1-5, **characterized in that** said prepurification is effected by means of one or more density gradients and/or via one or more prefiltrations.

7. The process according to claim 6, **characterized in that** one or more membrane filters are used in said prefiltration.

8. The process according to claim 7, **characterized in that** the membrane filter(s) for prepurification has/have a pore size of at least 250 nm.

9. The process according to any one of claims 1-8, **characterized in that** the pH of the virus-containing solution is 6-10, preferably 7.0-8.5, in particular 8.0.

10. The process according to any one of claims 1-9, **characterized in that** the virus-containing solution further contains protein.

11. The process according to claim 10, **characterized in that** said solution contains protein in the form of fetal calf serum or serum albumin.

12. The process according to claim 10 or 11, **characterized in that** the proportion of protein is not more than 30 % (v/v), preferably 15 % (v/v), in particular 10 % (v/v).

13. The process according to any of claims 1-12, **characterized in that** approx. 2-5 ml of solution per 1 cm² of filter surface, in particular not more than 2-2.5 ml of solution per 1 cm², is filtered.

## Revendications

1. Procédé pour la séparation quantitative de virus de diverses dimensions, **caractérisé en ce que** 1 à 10 ml d'une solution contenant un virus sont filtrés par cm² de surface filtrantes, **en ce que** la solution contenant le virus est filtré sur une ou plusieurs membranes filtrantes, **en ce que** la différence de dimension des virus à séparer est d'au moins 40 nm, **en ce que** les virus présentent une forme pratiquement sphérique, plus particulièrement une symétrie ikosaedrique et **en ce que** la réduction de titre des virus à séparer atteint une valeur supérieure à log 9.

2. Procédé selon la revendication 1, **caractérisé en ce que** la membrane filtrante est formée de fluorure de polyvinylidène, polytétrafluorométhylène, polypropylène, polyéthersulfone modifiée ou non modifiée, acétate de cellulose, nitrate de cellulose, polyamide ou cellulose régénérée, plus particulièrement cellulose régénérée par le coprammonium, de préférence le fluorure de polyvinylidène.

3. Procédé selon une des revendications 1 ou 2, **caractérisé en ce que** la ou les membrane(s) filtrante(s) présente/présentent une dimension de pores de l'ordre de 25 à 60 nm.

4. Procédé selon une des revendications 1-3, **caractérisé en ce que** les virus sont choisis dans le groupe comprenant AAV, les adénovirus ou les virus de l'herpés.

5. Procédé selon une quelconque des revendications 1-4, **caractérisé en ce que** la solution contenant un virus subit une purification préalable.

6. Procédé selon une quelconque des revendications 1 à 5, **caractérisé en ce que** la purification préalable est réalisée sur un ou plusieurs gradients de densité et/ou par une ou plusieurs filtrations préalables.

7. Procédé selon la revendication 6, **caractérisé en ce que** dans la filtration préalable précitée sont employés un ou plusieurs filtres membranaires.

8. Procédé selon la revendication 7, **caractérisé en ce que** le ou les filtre(s) membranaire(s) utilisé(s) pour la filtration préalable a/ont une distance de la pore au minimum de 250 nm.

9. Procédé selon une quelconque des revendications 1-8, **caractérisé en ce que** le pH de la solution contenant le virus est compris entre 6 et 10, de préférence 7,0 à 8,5, plus particulièrement est de 8,0.

10. Procédé selon une quelconque des revendications 1 à 9, **caractérisé en ce que** la solution contenant le virus contient en outre une protéine.

11. Procédé selon la revendication 10, **caractérisé en ce que** solution contenant le virus contient une protéine sous forme de sérum foetal de veau ou de sérumalbumine.

12. Procédé selon la revendication 10 ou 11, **caractérisé en ce que** la teneur en protéine n'est pas supérieure à 30% (v/v), de préférence à 15% (v/v), plus particulièrement est de 10% (v/v).

13. Procédé selon une quelconque des revendications 1 à 12, **caractérisé en ce que** sont filtrés environ 2 à 5 ml de solution par cm² de surface filtrante, plus particulièrement pas plus de 2 à 2,5 ml de solution par cm².
